# EUROPÄISCHE PATENTSCHRIFT

(11) **EP 1 226 799 B1**
(45) Veröffentlichungstag und Bekanntmachung des Hinweises auf die Patenterteilung: **04.05.2005**
(21) Anmeldenummer: 02000214.3
(22) Anmeldetag: 11.01.2002
(51) Int. Cl.: A61F 2/38

(54) **Inlay für eine Knie-Endoprothese**
Inlay for a knee endoprosthesis
Insert pour une endoprothèse du genou

(30) Priorität: 19.01.2001 DE 20100962 U
(43) Veröffentlichungstag der Anmeldung: 31.07.2002
(73) Patentinhaber: Mathys AG Bettlach, 2544 Bettlach (CH)
(72) Erfinder: Glien, Wilfried, 07639 Bad Klosterlausnitz (DE); Salomon, Dirk, 04626 Jonaswalde (DE); Katzer, Thomas, 07747 Jena (DE); Rahm, Jens, 08412 Werdau (DE); Thon, Klaus, 07629 Hermsdorf (DE)
(74) Vertreter: Körfer, Thomas, Dipl.-Phys.

(56) Entgegenhaltungen:
- EP-A- 0 765 645
- US-A- 6 004 351
- US-A- 6 013 103
- PATENT ABSTRACTS OF JAPAN vol. 1998, no. 11, 30. September 1998 (1998-09-30) & JP 10 155824 A (KYOCERA CORP), 16. Juni 1998 (1998-06-16) & JP 10 155824 A 16. Juni 1998 (1998-06-16)

## Beschreibung

Die Erfindung betrifft einen Meniskus-Komponente (Inlay) eines Tibia-Gelenkersatzes einer Knie-Total-Endoprothese, wobei die Meniskus-Komponente zwischen einer Tibiaplatte und einer Femurkondyle vorgesehen ist und eine konkav geformte Oberseite aufweist, die beim Beugen des Knies als Gleitlager für die Femurkondyle fungiert.

Solche Inlays sind aus EP-A-0 732 092 und EP-A-0 765 645 bekannt. Die konkave Ausbildung der Oberseite des Inlays erstreckt sich dabei über die gesamte Länge des Inlays und endet anterior und posterior in einer spitzwinkligen Kante (Lippung). Zwischen der Kondyle und dem Inlay findet eine kombinierte Gleit-Roll-Bewegung statt. Beim Beugen des Knies verschiebt sich der Berührungspunkt zwischen Kondyle und Inlay daher zum posterioren Ende. Die von der Kondyle auf das Inlay ausgeübte Kraft wirkt als Normalkraft im wesentlichen senkrecht zur Tangente im Berührungspunkt. Liegt dieser Berührungspunkt im posterioren Bereich des Inlays so ist diese Kraft posterior gerichtet und übt ein Kippmoment auf das Inlay aus, so dass das Inlay vorne angehoben wird und nach hinten kippt. Dies führt zu einer Luxation. Ein solches Kippmoment kann auch durch einen nicht optimalen Zustand des Bandapparats verursacht werden.

Diese Druckschriften betreffen ungekoppelte Knie-Prothesen, bei denen das Inlay nicht fest mit dem Tibiaplateau verbunden ist, sondern ihm gegenüber eine begrenzte Translations- und Rotationsbewegung ausführen kann und die Femurkondyle nur durch die konkave Form der Inlayoberseite und den natürlichen Bandapparat einschließlich des hinteren Kreuzbandes gehalten wird, so dass sich die Femurkondyle anterior-posterior gegenüber dem Inlay verschieben kann.

Aus DE-A-43 08 563 ist es bekannt, das Inlay mittels einer Schwalbenschwanzführung auf dem Tibiaplateau festzulegen, so dass zwar eine Translations- und Rotationsbewegung möglich ist, das Inlay jedoch nicht von den Tibiaplateau abheben kann. Eine entsprechend ausgebildete Führung ist auch aus EP-A-0 634 156 bekannt. Diese Zwangsführung stellt einen sog. passiven Kippschutz dar. Das Inlay besteht im allgemeinen aus Polyethylen, während das Tibiaplateau aus einer Titanlegierung oder CoCrMo-Legierung besteht. Beim Auftreten eines Kippmoments entsteht in dieser Zwangsführung zusätzlicher Abrieb an dem PE-Inlay oder in Folge hoher Flächenpressung sehr kritischer Kaltfluss an UHMW-PE.

US-A 6,004,351 beschreibt ein prothetisches Kniegelenk gemäß dem Oberbegriff von Anspruch 1, welches eine großwinklige Krümmung bzw. Beugung des Knies ermöglicht. Das dort beschriebene Kniegelenk weist eine femorale Komponente auf, die angepasst ist, um an einem distalen Femur befestigt zu werden. Weiterhin weist das prothetische Kniegeienk eine tibiale Komponente auf, die angepasst ist, um an einer proximalen Tibia befestigt zu werden. Zwischen der femoralen und tibialen Komponente ist ein Inlay aus Polyethylen gleitend zwischengelagert. Dieses Inlay weist eine obere konkave Oberfläche auf, welche die Lagerfläche der femoralen Komponente trägt. Weiterhin weist das Inlay eine konvexe untere Oberfläche auf. Die konkave Oberfläche ist mit einen Krümmungsradius von 20 mm bis 1000 mm auf der gesamten Oberfläche zwischen dem anterioren und dem posterioren Ende versehen.

Der Erfindung liegt die Aufgabe zugrurde, ein Inlay zu schaffen, bei dem die Gefahr des Kippens des Inlays und damit eine Luxation des Kniegelenkes vermieden wird, ohne dass ein zusätzlicher Abrieb oder Kaltfluss an dem Inlay in Kauf genommen werden muss.

Erfindungsgemäß wird diese Aufgabe durch ein Inlay mit den Merkmalen gemäß Anspruch 1 gelöst. Vorteilhafte Weiterbildungen sind in den abhängigen Ansprüchen definiert. Gemäß einer bevorzugten Ausführungsform kann der konvexe Bereich der Oberseite des Inlays etwa das posteriore Viertel des Inlays umfassen. Erfindungsgemäß sind der Krümmungsmittelpunkt und radius des konvexen Bereichs so bemessen, dass der Scheitelpunkt der konvexen Krümmung 10mm vor dem posterioren Ende oder maximal 2 mm dahinter liegt. Vorzugsweise ist die Oberseite des Inlays am posterioren Ende horizontal. Vorzugsweise beträgt der Krümmungsradius etwa 10 bis 14 mm und liegt der Krümmungsmittelpunkt etwa 2 mm vor dem posterioren. Ende des Inlays.

Vorzugsweise ist der konvexe Bereich der Oberseite des Inlays so geformt, dass bei radialer Krafteinleitung der Schnittpunkt des Kraftvektors mit der Inlayauflagefläche stets ventral des posterioren Inlayrandes liegt.

Der konkave, als Gleitlager für die Kondyle fungierende Bereich der Oberseite des Inlays hat entsprechend dem natürlichen Knie eine laterale und mediale Mulde und die Kondyle ist dementsprechend mit einem lateralen und medialen Kondylenelement ausgebildet, die in der entsprechenden Mulde rollen und gleiten. Die Mulden sind sowohl sagittal als auch medial-lateral konkav gekrümmt. In dem konvexen posterioren Bereich der Oberseite des Inlays bleibt die medial-laterale Krümmung erhalten und ist nur in Sagittalrichtung eine konvexe Krümmung vorhanden, so dass insgesamt eine Sattelfläche entsteht.

Beim erfindungsgemäßen Inlay ist somit keine ausgeprägte posteriore Lippung vorhanden. Durch die konvexe Krümmung des posterioren Bereichs der Inlayoberseite ist die von der gebeugten Femurkondyle auf den posterioren Bereich des Inlays ausgeübte Normalkraft so gerichtet, dass sie durch die Unterseite des Inlays und nicht durch den umlaufenden vertikalen Rand zeigt, so dass auf das Inlay kein Kippmoment ausgeübt wird (aktiver Kippschutz).

Das Inlay kann mobil (gegenüber dem Tibiaplateau beweglich) oder verriegelt (am Tibiaplateau fixiert) sein.

Ein verriegeltes Inlay kann zwar nicht kippen, das dennoch vorhandene Kippmoment wird nun jedoch in die Verbindungsfläche Knochen-Tibiaplateau übertragen und erzeugt unter ungünstigen Bedingungen Zugkräfte in diesem Bereich. Solche Zugkräfte begünstigen oder provozieren eine Auslockerung des tibialen Implantats in der Tibia. Derartige Kippmomente können bei der erfindungsgemäß konvexen Ausformung des Inlays nicht entstehen.

Das erfindungsgemäße Inlay besteht aus UHMW-PE und wird im allgemeinen in Verbindung mit einem Tibiaplateau aus einer bioverträglichen Titan- oder CoCrMo-Legierung oder einem bioverträglichen Keramikmaterial eingesetzt.

Ein Kippen eines mobilen Inlays könnte zwar auch durch eine Zwangsführung des Inlays an dem Tibiaplateau verhindert werden, z.B. durch eine Schwalbenschwanzführung. Ein Bewegen des Inlays gegenüber dem Tibiaplateau bei bestehendem Kippmoment oder bei seitlichen Kräften führt jedoch zu einem erhöhten Verschleiß innerhalb der Führung und damit zu einem unerwünschten Abrieb des PE-Materials des Inlays. Infolge des erfindungsgemäßen aktiven Kippschutzes ist eine Zwangsführung des Inlays auf dem Tibiaplateau nicht erforderlich.

Ein Ausführungsbeispiel der Erfindung wird nachfolgend anhand der Zeichnung näher erläutert. Es zeigen:
- Fig. 1: im Schnitt die wesentliche Bestandteile der Knie-Endoprothese bei gestrecktem Knie;
- Fig. 2: einen Schnitt ähnlich dem von Fig. 1, bei gebeugtem Knie;
- Fig. 3: das Entstehen des Kippmoments bei einem herkömmlichen Inlay;
- Fig. 4: die bei dem erfindungsgemäßen Inlay zwischen Kondyle und Inlay in dessen posterioren Bereich auftretende Kraft, die kein Kippmoment erzeugt, und
- Fig. 5: ein Detail des hinteren Endbereichs des Inlays.

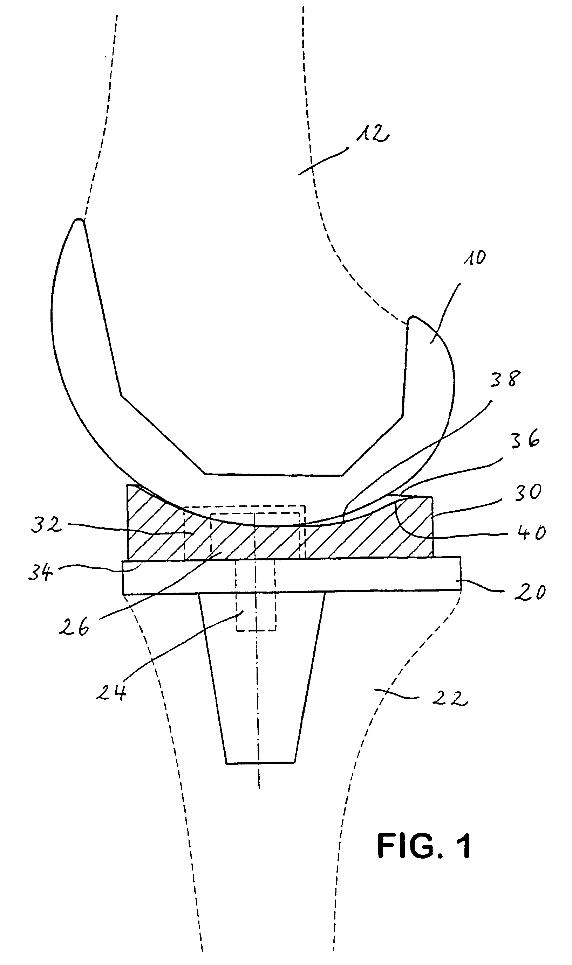

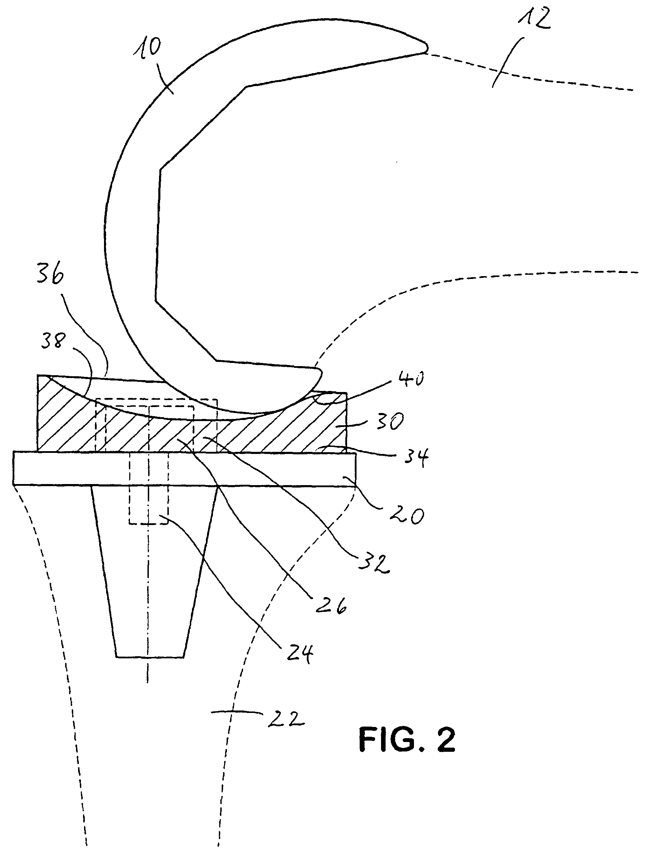

Fig. 1 und 2 zeigen eine Totalendoprothese mit einer Femurkondyle 10, einem Tibiaplateau 20 und dazwischen einem als Meniskus-Komponente fungierenden Inlay 30. In Fig. 1 ist die Endoprothese bei gestrecktem Knie und in Fig. 2 bei gebeugtem Knie dargestellt. Die Kondyle 10 wird am Femur 12 verankert und das Tibiaplateau 20 wird an der Tibia 22 verankert, wobei verschiedene Verankerungstechniken bekannt sind.

Das sich dazwischen befindende Inlay 30 kann in Abhängigkeit vom Aufbau der Prothese in einem vorgegebenen Ausmaß gegenüber dem Tibiaplateau 20 gleiten und rotieren (bewegliches Inlay) oder mit ihm fest verbunden sein (verriegeltes Inlay).

Von oben betrachtet hat das Tibiaplateau 20 Nieren-Form mit einer halbkreisförmigen posterioren Aussparung für das hintere Kreuzband. Das in der Zeichnung dargestellte bewegliche Inlay 30 ist scheibenförmig und hat von oben betrachtet eine ähnliche Form wie das Tibiaplateau 20, doch etwas kleinere Abmessungen mit ebenfalls einer posterioren Aussparung für das hintere Kreuzband. In der Mitte des Tibiaplateaus 20 ist ein Gewindebolzen 24 eingeschraubt, dessen zylindrischer Kopf 26 vorsteht. Das Inlay 30 hat in der Unterseite 34 eine längliche oder runde Ausfräsung 32, die den Kopf 26 mit Spiel aufnimmt. Die geometrischen Verhältnisse des Tibiaplateaus 20 zum Inlay 30 und des Kopfes 26 zur Ausfräsung 32 sind so gewählt, dass auch bei einer Beugung der Kondyle (Fig. 2) das Inlay 30 frei, z.B. um ± 20°, gegenüber dem Tibiaplateau 20 rotieren kann und anterior-posterior etwa 9 mm und medial-lateral etwa 2 mm gleiten kann, ohne dass das Inlay 30 über das Tibiaplateau 20 übersteht.

Wird das Inlay fest mit dem Tibiaplateau 20 verbunden, so findet eine Roll-Gleitbewegung, d.h. Walkbewegung, zwischen Inlay und Kondyle statt. Bei einem beweglichen Inlay finden dagegen Gleitbewegungen in zwei Ebenen statt, nämlich zum einen zwischen Tibiaplateau 20 und Inlay 30 und zum anderen zwischen Inlay 30 und Kondyle 10. Walkbewegungen zwischen Inlay 30 und Kondyle 10 werden dadurch minimiert, wodurch sich die Lebensdauer des Implantats erhöht.

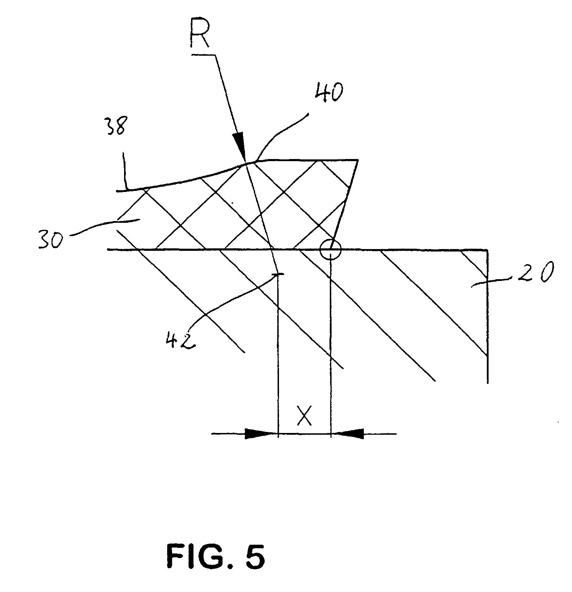

Die Oberseite 36 des Inlays 30 hat eine mediale und eine laterale konkave, flache Vertiefung oder Mulde 38 mit einer Eminentia dazwischen. In den Mulden 38 rollen und/oder gleiten die Femurkondylen 10, wenn das Knie gebeugt oder gestreckt wird. Die Mulden 38 gehen in ihrem posterioren Bereich in eine konvexe Gleitfläche 40 über. Die konvexe Gleitfläche 40 erstreckt sich etwa über ein Fünftel bis ein Viertel der Abmessung des Inlays 30 in anterior-posteriorer Richtung. Wie in Fig. 5 ersichtlich, beträgt der Krümmungsradius R der konvexen Gleitfläche 40 etwa 10 bis 14 mm und liegt der Krümmungsmittelpunkt 42 der konvexen Gleitfläche 40 etwa 2 mm (Strecke X) vor dem posterioren Ende des Inlays 30. In medial-lateraler Richtung sind die Gleitflächen weiterhin schwach konkav gekrümmt.

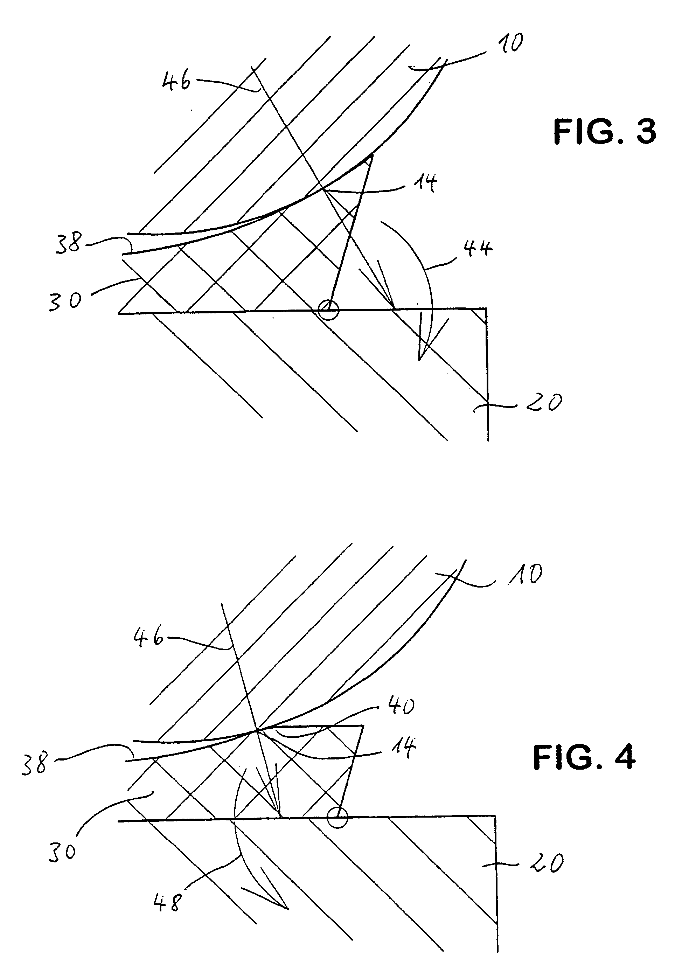

In Fig. 3 ist im Schnitt ein Inlay 30 mit einer vollständig konkaven Oberseite 36 gezeigt, wie es bisher üblich war, während in Fig. 4 das erfindungsgemäße Inlay 30 mit posterior konvex auslaufender Mulde 38 dargestellt ist.

Die von der Kondyle 10 auf das Inlay 30 ausgeübte Kraft 46 zeigt im wesentlichen immer normal zur Oberfläche des Inlays 30, d.h. rechtwinklig zur Tangente im Berührungspunkt 14. Bei Beugung des Knies kann sich die Kondyle 10 je nach der postoperativen Bandsituation nach posterior verschieben, so dass der Berührungspunkt zwischen der Kondyle 10 und dem Inlay 30 am posterioren Ende des Inlays 30 liegt. Bei der ausschließlich konkaven Vertiefung von Fig. 3 hat diese Normalkraft 46 eine posterior gerichtete Komponente, die um so größer ist, je weiter posterior der Berührungspunkt 14 zwischen Kondyle 10 und Inlay 30 liegt. Infolge des posterior gelegenen Berührungspunktes 14 zwischen den Kondylen 10 und dem Inlay 30 und der posterior gerichteten Normalkraft 46 verläuft der Kraftvektor durch den hinteren Rand des Inlays, so dass ein Kippmoment 44 entsteht, d.h. die Vorderseite des Inlays 30 wird angehoben. Als Folge kann die Kondyle 10 vom hinteren Ende des Inlays 30 abrutschen und kann es zu einer Luxation kommen.

Bei dem erfindungsgemäß ausgebildeten Inlay 30 von Fig. 4 verläuft der Vektor der Normalkraft auch dann, wenn der Berührungspunkt 14 zwischen der Kondyle 10 und dem Inlay 30 am posterioren Ende des Inlays 30 liegt, immer durch die Unterseite 34 des Inlays 30, so dass kein Kippmoment entsteht sondern ein in entgegengesetzter Richtung zeigendes Drehmoment 48. Dies wird durch die konvexe Ausbildung des hinteren Endes der Oberseite 36 des Inlays 30 erreicht, wobei der Krümmungsmittelpunkt der konvexen Gleitfläche 40 vorzugsweise innerhalb der nach unten gerichteten Projektion des Inlays 30, d.h. der Auflagefläche oder Unterseite des Inlays 30 liegt, und allenfalls 2 mm hinter dem posterioren Ende des Inlays liegt. Der Vektor der von der Kondyle 10 auf das Inlay 30 ausgeübten Kraft durchstößt daher die Unterseite des Inlays 30, so dass kein Kippmoment entsteht. Durch die konvexe Gleitfläche 40 wird daher die Kippsicherheit des Inlays 30 bei gebeugter Kondyle 10 gewährleistet.

Das Inlay 30 kann auch verriegelt sein, wobei die Oberseite des Tibiaplateaus 20 dann z.B. anterior eine nach vorne offene V-förmige Einfräsung hat und ansonsten plan ist. Auch ein solches Inlay ist scheibenförmig und hat von oben betrachtet eine ähnliche Form wie das Tibiaplateau mit ebenfalls einer posterioren Aussparung für das hintere Kreuzband. Die Abmessungen eines verriegelten Inlays entsprechen im Allgemeinen den des Tibiaplateaus. Die Unterseite 34 des Inlays 30 ist eben und hat anterior eine nach unten zeigende Nase, die in der V-förmigen Einfräsung des Tibiaplateaus 20 liegt. Das Inlay 30 kann auf dem Tibiaplateau mittels eines Gewindebolzens, der durch ein Langloch in dem Inlay geht weitgehen festgelegt sein, so dass es keine Rotation und nur eine sehr begrenzte sagittale Verschiebung ausführen kann. Die Beweglichkeit des Inlays kann auch durch einen erhöhten umlaufenden Rand des Tibiaplateaus eingeschränkt sein. Auch bei solchen verriegelten Inlays ist die Vermeidung von Kippkräften von Vorteil.

### Bezugszeichenliste

- 10: Kondyle
- 12: Femur
- 14: Berührungspunkt
- 20: Tibiaplateau
- 22: Tibia
- 24: Gewindebolzen
- 26: Kopf
- 30: Inlay
- 32: Ausfräsung
- 34: Unterseite
- 36: Oberseite
- 38: Mulde
- 40: konvexe Gleitfläche
- 42: Scheitelpunkt
- 44: Kippmoment
- 46: Normalkraft
- 48: Drehmoment

## Patentansprüche

1. Inlay für eine Knie-Totalendoprothese, wobei das Inlay (30) als Meniskuskomponente zwischen einer Tibiaplatte (20) und einer Femurkondyle (10) vorzusehen ist und eine konkav geformte Oberseite (36) aufweist, die beim Beugen des Knies als Gleitlager für die Femurkondyle (10) fungiert, wobei der posteriore Bereich der Oberseite (36) des Inlays (30) im Sagittalschnitt konvex geformt ist,
**dadurch gekennzeichnet,**
**dass** der Krümmungsmittelpunkt des konvexen Bereichs (40) im Bereich von 10 mm vor bis 2 mm hinter dem posterioren Ende der Unterseite des Inlays (30) liegt.

2. Inlay nach Anspruch 1,
**dadurch gekennzeichnet,**
**dass** der konvexe Bereich (40) der Oberseite (36) etwa das posteriore Viertel des Inlays (30) umfasst.

3. Inlay nach einem der vorherigen Ansprüche,
**dadurch gekennzeichnet,**
**dass** der Scheitelpunkt (42) des konvexen Bereichs (40) etwa 14 bis 6 mm vor dem posterioren Ende des Inlays (30) liegt.

## Claims

1. Inlay for a total knee replacement, wherein the inlay (30) is to be provided as a meniscus component between a tibial plate (20) and a femoral condyle (10) and has a concave-shaped upper side (36), which during bending of the knee acts as a sliding bearing for the femoral condyle (10), wherein the posterior region of the upper side (36) of the inlay (30) in sagittal section is convex-shaped,
**characterized in**
**that** the centre of curvature of the convex region (40) lies in the range between 10 mm in front of and 2mm behind the posterior end of the underside of the inlay (30).

2. Inlay according to claim 1,
**characterized in**
**that** the convex region (40) of the upper side (36) comprises approximately the posterior quarter of the inlay (30).

3. Inlay according to one of the preceding claims,
**characterized in**
**that** the vertex (42) of the convex region (40) lies approximately 14 to 6 mm in front of the posterior end of the inlay (30).

## Revendications

1. Insert pour une endoprothèse totale du genou, dans lequel l'insert (30) est prévu en tant que composant du ménisque entre une plaque tibiale (20) et un condyle du fémur (10) et présente un côté supérieur (36) de forme concave qui, lors de la flexion du genou fait office de palier coulissant pour le condyle du fémur (10), dans lequel la partie postérieure du côté supérieur (36) de l'insert (30) présente une forme convexe en coupe sagittale,
**caractérisé en ce que**,
le point médian de courbure de la région convexe (40) se situe dans une plage de 10 mm devant jusqu'à 2 mm derrière l'extrémité postérieure du côté inférieur de l'insert (30).

2. Insert selon la revendication 1,
**caractérisé en ce que**,
la région convexe (40) du côté supérieur (36) renferme approximativement le quart postérieur de l'insert (30).

3. Insert selon l'une des revendications précédentes,
**caractérisé en ce que**,
le point apical (42) de la région convexe (40) se situe à environ 14 jusqu'à 6 mm devant l'extrémité postérieure de l'insert (30).
